# EUROPEAN PATENT APPLICATION

(11) **EP 4 442 248 A1**
(43) Date of publication of application: **09.10.2024**
(21) Application number: 23166885.6
(22) Date of filing: 05.04.2023
(51) Int. Cl.: A61K 9/00, A61K 31/7004, A61P 31/00

(54) **COMPOSITIONS FOR USE IN TREATING INFECTIONS**

(71) Applicant: PathoBlock Therapeutics GmbH & Co. KG, 10117 Berlin (DE)
(72) Inventor: Schumacher, Udo, 22085 Hamburg (DE); Hänsch, Inken Lena, 10179 Berlin (DE)
(74) Representative: Uexküll & Stolberg

(57) **Abstract**

The present invention provides compositions comprising D-galactose and L-fucose for use in treating or preventing infections in an individual, wherein the composition is administered to the individual in solid form, preferably as a powder. The present invention further provides compositions comprising D-galactose and L-fucose for use in treating or preventing infections in an individual, wherein the composition is administered to the individual in liquid form and wherein the liquid composition is produced by mixing D-galactose and L-fucose in solid form with a liquid no longer than 20 hours before administering the composition to the individual.

## Description

### FIELD OF THE INVENTION

The present invention relates to compositions comprising D-galactose and L-fucose for use in treating or preventing infections in an individual. In particular, the present invention relates to improved pharmaceutical compositions comprising D-galactose and L-fucose for treating or preventing infections in an individual.

### BACKGROUND OF THE INVENTION

*Pseudomonas aeruginosa* is a common bacterium that usually occurs in hospitalized patients in intensive care units or in patients with suppressed immune systems and is particularly dangerous for patients with chronic lung diseases. *Pseudomonas aeruginosa* causes severe and often deadly infections such as pneumonia, wound, external auditory canal and bloodstream infections. While infections with this pathogen are often severe and life threatening, the only treatment option so far are antibiotics. Antibiotic therapy is known to cause a range of side effects. Even worse, due to increasing antibiotic resistances, the World Health Organization listed *Pseudomonas aeruginosa* as one of the top three candidates that need novel therapies. There are no effective treatment options for antibiotic-resistant *Pseudomonas aeruginosa* infections, which is why alternative treatment strategies are urgently needed.

*Pseudomonas aeruginosa* infects human cells by secreting two lectins, lectin A (PA-IL or Lec A) and lectin B (PA-IIL or Lec B) (Gilboa-Garber (1982) Methods Enzymol. 83:378-3) that bind to specific sugars of the glycocalyx and those of the extracellular matrix glycoproteins thereby facilitating the infection process (Gilboa-Garber and Garber (1989) FEMS Microbiol Rev. 5:211-211; Adam et al. (1997) Am J Respir Crit Care Med. 155:2102-2104). Lectin A is specific for D-galactose, while lectin B is specific for L-fucose. Both lectins also inactivate the beating of the cilia in the airways, which facilitates spreading of *Pseudomonas aeruginosa* in the airways and lungs (Adam et al. (1997) Am J Respir Crit Care Med. 155:2102-2104, Mewe et al. (2005) J Laryngol Otol. 119(8):595-9). Both lectins further contribute to quorum sensing and biofilm generation of the bacterium (Winzer et al. (2000) J Bacteriol. 182:6401-6411), demonstrating the importance of these pathogenicity factors.

Blocking the adhesion of *Pseudomonas aeruginosa* by external addition of specific sugars has been tested and represents an alternative to standard antimicrobial therapies. If unable to attach to human cells and matrices via the lectins, the pathogens are removed by non-specific anti-microbial mechanisms. It has been shown that this approach also works in the clinic (von Bismarck et al. (2001) Klin Padiatr. 213:285-287; Hauber et al (2008) Int J Med Sci. 5:371-376). For this purpose, the two specific sugars, D-galactose and L-fucose, are commonly administered in the form of aqueous solutions.

However, this form of therapy has not been broadly used, nor achieved regulatory approval or commercial acceptance. This could be due to the fact that aqueous compositions comprising D-galactose and L-fucose become ineffective over time.

The sugar-specific lectins can distinguish between the alpha- and beta-configuration of the sugars (Ghazarian et al. (2011) Acta Histochem. 113:236-247). When sugar in solid form is dissolved in a solution, a thermodynamic equilibrium between the alpha-, beta- and linear configuration of the sugar is established over time.

There is therefore still a need for improved pharmaceutical compositions comprising D-galactose and L-fucose and for the use thereof in the treatment of infections in an individual, in particular for treating and preventing *Pseudomonas aeruginosa* infections in an individual.

### SUMMARY OF THE INVENTION

In one aspect, the invention provides compositions comprising D-galactose and L-fucose for use in treating or preventing infections in an individual, wherein the composition is administered to the individual in solid form, preferably as a powder.

In a further aspect, the present invention provides compositions comprising D-galactose and L-fucose for use in treating or preventing infections in an individual, wherein the composition is administered to the individual in liquid form and wherein the liquid composition is produced by mixing D-galactose and L-fucose in solid form with a liquid to obtain the composition no longer than 20 hours before administering the composition to the individual.

The present invention also provides compositions comprising D-galactose for use in treating or preventing infections in an individual, wherein treating comprises the administration of D-galactose and L-fucose in solid form, preferably as a powder, or as a liquid composition produced no longer than 20 hours before administering the composition to the individual.

In a related aspect the invention provides compositions comprising L-fucose for use in treating or preventing infections in an individual, wherein treating comprises the administration of D-galactose and L-fucose in solid form, preferably as a powder, or as a liquid composition produced no longer than 20 hours before administering the composition to the individual.

The above compositions for use in treating or preventing infections in an individual according to the invention preferably contain D-galactose in the beta form and L-fucose in the alpha form.

The present invention also provides a container comprising a solid composition as described above, preferably a powder, and an opening for administering the solid composition to a patient.

In one aspect a respective container comprises two compartments and a means for dispensing the content of each compartment, wherein one compartment comprises a liquid and the other compartment comprises a solid composition as described above, and wherein the container comprises a means for mixing the solid composition with the liquid.

In a further embodiment, the present invention provides a dressing, bandage or plaster comprising a composition as described above, which can be directly applied to the infected site.

Further, the invention also provides a device such as an inhaler or nebulizer to deliver a composition as described above to the lungs of a patient.

### DETAILED DESCRIPTION

The present invention provides compositions comprising D-galactose and L-fucose for use in the treatment or prevention of infections in an individual, wherein composition is in solid form, such as a powder, or wherein the composition is a liquid composition that is prepared by mixing D-galactose and L-fucose in solid form with a liquid to obtain the composition no longer than 20 hours before administering the composition to the individual.

Without being bound to theory, the present invention is based on the observation that in aqueous solutions D-galactose and L-fucose undergo mutarotation which quickly converts a significant amount of the sugars into a non-binding conformation. Mutarotation therefore decreases the effective concentration of each sugar in the specific configuration. Consequently, prior art compositions for treating microbial infections comprising D-galactose and L-fucose were partially ineffective and in fact inactivated upon storage.

Insofar as the present invention relates to compositions comprising D-galactose and L-fucose for use in the treatment or prevention of infections in an individual, wherein the composition is a liquid composition that is prepared by mixing D-galactose and L-fucose in solid form with a liquid to obtain the composition the same is prepared no longer than 20 hours before administering the composition to the individual, preferably no longer than 12 hours, no longer than 6 hours, no longer than 3 hours, no longer than 1 hour or no longer than 30 minutes before administering the composition to the individual.

The compositions for use in treating or preventing infections in an individual according to the present invention can be administered using any concentration of D-galactose to L-fucose. Preferably the relative concentration of D-galactose to L-fucose is in the range of 1:10000 to 10000:1, preferably 1:1.

Insofar as the compositions for use in treating or preventing infections in an individual according to the invention are solid compositions, the quantity of D-galactose is preferably in the range of 0.001 mg to 10 g per cm² application site and the quantity of L-fucose is in the range of 0.001 mg to 10 g per cm² application site, preferably the quantity of D-galactose in the solid composition is 0.02 g per cm² application site and the quantity of L-fucose in the solid composition is 0.02 g per cm² application site.

Insofar as the compositions for use in treating or preventing infections in an individual according to the invention are inhaled, the composition is in solid form and the dose of D-galactose per inhalation is in the range of 0.001 mg to 5.0 g and the dose of L-fucose per inhalation is in the range of 0.001 mg to 5.0 g, preferably the dose of D-galactose per inhalation is 0.1 g and the application dose of L-fucose per inhalation is 0.1 g. In this embodiment it is further preferred that the solid consists of particles smaller than 50 µm, preferably smaller than 30 pm, such as smaller than 15 pm, and most preferred smaller than 10 µm.

Insofar as the compositions for use in treating or preventing infections in an individual according to the invention are liquid compositions, the concentration of D-galactose in the liquid composition is in the range of 0.001 mM to 3.0 M and wherein the concentration of L-fucose in the liquid composition is in the range of 0.001 mM to 3.0 M, preferably the concentration of D-galactose in the liquid composition is 0.15 M and the concentration of L-fucose in the liquid composition is 0.15 M.

The compositions can be used for treating or preventing infections in general, in particular bacterial infections and most preferred an infection suspected to be caused by a microorganism expressing L-fucose- or D-galactose-binding lectins, such as *Pseudomonas aeruginosa.*

In one aspect, the compositions for use in treating or preventing infections in an individual according to the invention are administered topically.

Different infections can be treated with the compositions according to the invention and include respiratory tract infection, for example in patients suffering from cystic fibrosis, ear nose throat infection, for example otitis externa, skin infection, dermatitis, wound infection, urinary tract infection, catheter-related infection, osteomyelitis, implant-associated infection, eye infection, surgical site infection or joint infection.

As already indicated, the core aspect of the invention resides in transferring D-galactose and L-fucose shortly before administration from a stable aggregate state or form (solid) to an unstable aggregate state or form (dissolved in a liquid) of administration, i.e., shortly before or during administration to the patient.

In particular the compositions according to the invention can be administered as a powder, an aerosol, a liquid composition of varying viscosities, for example a gel or an ointment, or wherein administration of the composition comprises inhaling the composition.

In a further embodiment the present invention provides a container comprising a solid composition as described above, preferably a powder, and an opening for administering the solid composition to a patient.

An alternative container provided by the present invention can comprise two compartments and a means for dispensing the content of each compartment, wherein one compartment comprises a liquid and the other compartment comprises a solid composition according to any one of the preceding claims, and wherein the container comprises a means for mixing the solid composition with the liquid. These containers are used in a method comprising mixing D-galactose and L-fucose with the liquid no longer than 20 hours before administration or during administering the composition.

The present invention also provides dressings, bandages or plasters comprising a composition as described above, which can be directly applied to the infected site.

Finally, the present invention provides devices such as an inhaler or nebulizer to deliver a composition as described above to the lungs of a patient.

### DEFINITIONS

Unless otherwise noted, terms are to be understood according to conventional usage by those of ordinary skill in the relevant art.

As used herein, the term "sugar" includes soluble carbohydrates such as monosaccharides like glucose, fucose and galactose and disaccharides composed of two chemically bound monosaccharides by a glycosidic linkage like sucrose, lactose and maltose.

As used herein, the term "anomer" relates to the configuration at the anomeric centre of a sugar molecule and is termed alpha-(α-) or beta- (β-)anomer. It refers to the stereocentre that determines the absolute configuration of the biological molecule. The term refers to the overall three-dimensional structure of the carbohydrate that results from its basic molecular structure.

As used herein, "mutarotation" refers to the interconversion between different diastereomers (anomers) of a monosaccharide in aqueous solutions. In aqueous solutions monosaccharide generally exist as an equilibrium mixture of the different anomers.

The term "about" is used to indicate that a value includes the standard deviation of error for the device or method being employed to determine the value. The use of the term "or" in the claims is used to mean "and/or" unless explicitly indicated to refer to alternatives only or the alternatives are mutually exclusive, although the disclosure supports a definition that refers to only alternatives and to "and/or." When used in conjunction with the word "comprising" or other open language in the claims, the words "a" and "an" denote "one or more," unless specifically noted. The terms "comprise," "have" and "include" are open-ended linking verbs. Any forms or tenses of one or more of these verbs, such as "comprises," "comprising," "has," "having," "includes" and "including," are also open-ended. For example, any method that "comprises," "has" or "includes" one or more steps is not limited to possessing only those one or more steps and also covers other unlisted steps.

### DESCRIPTION OF THE FIGURES

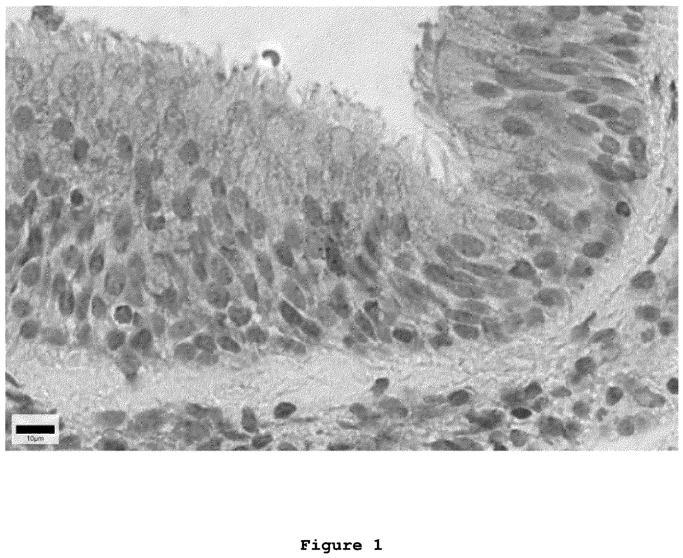

**Figure 1** demonstrates the human respiratory epithelia negative control at time point 0. No lectin was applied. No red staining is seen.

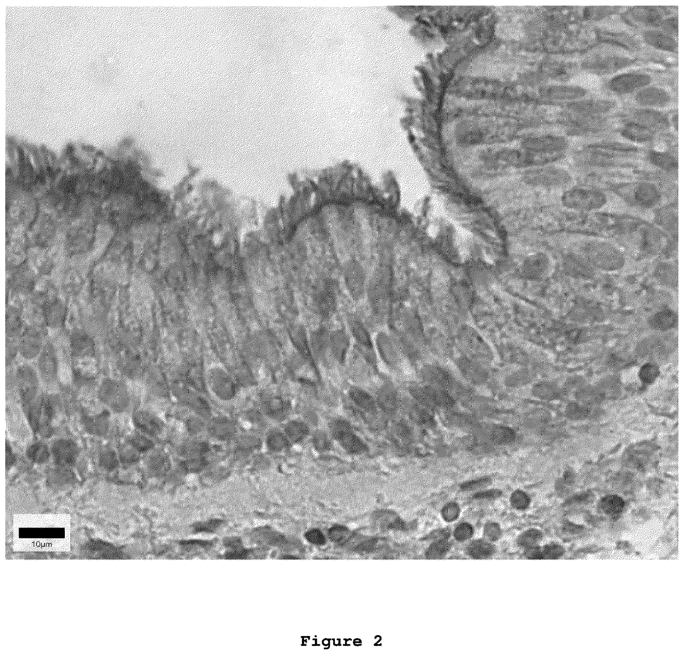

**Figure 2** shows the binding of PA-IIL to human respiratory epithelia at time point zero (0). 5 ug/ml PA-IIL was applied to the tissue. PA-IIL binding at the cilia can be seen by the significant amount of red coloration product.

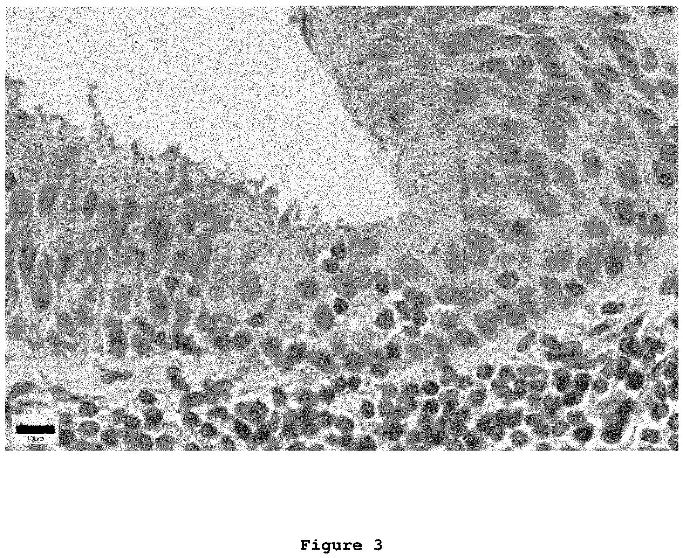

**Figure 3** shows the inhibition of PA-IIL binding to human respiratory epithelia by L-fucose at time point zero (0). 5 ug/ml PA-IIL was incubated with freshly dissolved 0.01 mM L-fucose. PA-IIL lectin binding at the cilia was abolished by L-fucose as demonstrated by the absence of a red coloration product.

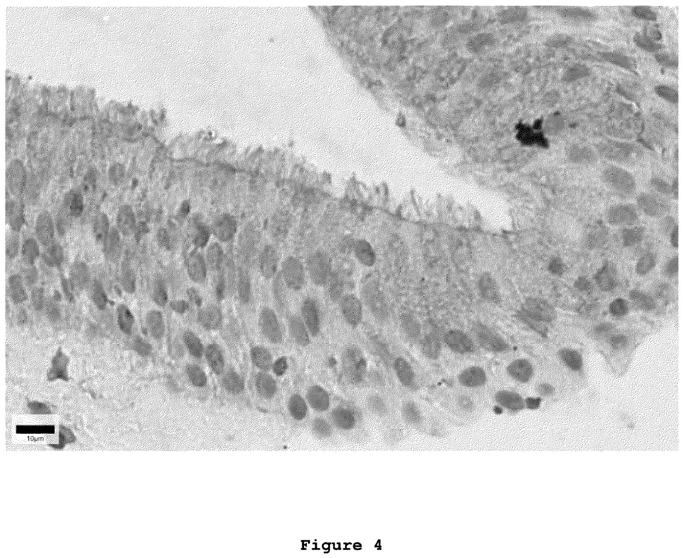

**Figure 4** shows the decreased inhibition of PA-IIL binding to human respiratory epithelia by L-fucose after 12 hours incubation. 0.01 mM L-fucose was dissolved and stored for 12 hours in lectin buffer and then incubated with 5 pg/ml PA-IIL. PA-IIL lectin binding at the cilia was not completely abolished by L-fucose as demonstrated by a faintly present red coloration product.

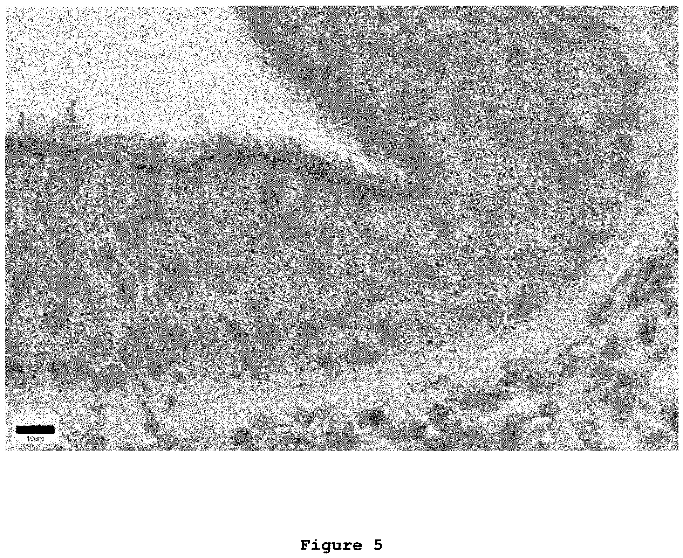

**Figure 5** shows no inhibition of PA-IIL binding to human respiratory epithelia by L-fucose after 24 hours incubation. 0.01 mM L-fucose was dissolved and stored for 24 hours in lectin buffer and then incubated with 5 pg/ml PA-IIL. PA-IIL lectin binding at the cilia was not abolished by L-fucose as demonstrated by the red coloration product.

### EXAMPLE

The following example further illustrates the present invention. It is for illustration only, and not meant to restrict the scope of the invention.

### Materials and methods

### Histochemical analysis of PA lectin binding to human respiratory epithelia

Tissue preparation and lectin histological staining has been described (Schreiber et al. (2014) Anticancer Res. 34(12) :7045-53; Böckelmann et al. (2021) Cell Oncol. (Dordr) 44(5) :1183-1195) . Five µm thick sections of human airway epithelium were applied to Adhesion Micro Slides (Histo Bond^{®}; Marienfeld GmbH, Lauda-Königshofen Germany). Slides were deparaffinized and rehydrated through a series of graded ethanol to distilled water. Lectin histochemistry was performed with and without compositions comprising L-fucose and biotinylated PA-IIL lectins (Mewe et al. (2005) J Laryngol Otol. 119(8):595-9). Deparaffinized sections were briefly incubated in lectin buffer (LB) consisting of tris-buffered saline (TBS contained Trizma Base, 50 mM Tris; Sigma, Steinheim, Germany), sodium chloride (150 mM; J.T. Baker, Deventer, the Netherlands) and hydrochloric acid (Merck, Darmstadt, Germany) in distilled water (adjusted to pH 7.6) with addition of 1% MgCl₂ and 1% CaCl₂ (Merck, Darmstadt Germany). Sections were then treated with 0.1% trypsin (Biochrom KG, Berlin, Germany) dissolved in LB and incubated for 10 min at 37°C. To stop the digestion of the sections with trypsin, the sections were washed in running tap water for 5 min. The sections were then washed three times (5 min each) in LB and incubated with and without a composition comprising L-fucose and biotinylated PA-IIL (5 pg/ml) for 30 min in a humid chamber at room temperature. This step was followed by three washes (5 min each) in TBS. Afterwards the sections were incubated with alkaline phosphatase streptavidin complex (Vectastain^{®} ABC-Kit; Vector Laboratories) in a humid chamber for 30 min. After final three rinsings (5 min each) in TBS, sections were transferred into the visualizing mixture containing naphthol-AS-biphosphate, hexatozised new fuchsin, dimethylformamide and tween 20 (all from Sigma-Aldrich Chemie, Darmstadt, Germany) applied in the dark for 20 min to visualize enzyme reactivity. To stop the enzyme reaction, the sections were washed in running tap water (7 min) and were then transferred into distilled water (2 min). Counterstaining was performed using Mayer's hemalum (Merck). The sections were dehydrated and subsequently covered using a resinous permanent mounting medium (Eukitt, Kindler GmbH, Freiburg, Germany). Slides were first evaluated using a ZEISS Axiophot 2 microscope (Carl Zeiss, Jena, Germany). Digital images were obtained with a ZEISS Axio Scan Z1 slide scanner equipped with a ZEISS EC Plan-Neofluar 20x/0,50 Pol M27 objective (Carl Zeiss, Jena, Germany) and a Hitachi HV-F20SCL camera with 1600 x 1200 pixels (Hitachi Kokusai Electric America Ltd., New York, USA). For image acquisition ZEISS ZEN 2.3 software was used (Carl Zeiss, Jena, Germany). Images were further processed with netScope Viewer software (Net-Base Software, Freiburg, Germany).

### Lectin Blocking Efficacy of Sugar Solutions in relation to storage time

For the lectin blocking experiments, 10 pg/ml PA-IIL solution dissolved in LB was incubated 1:1 with a freshly prepared L-fucose solution (Merck) for 30 min resulting in a final 5 pg/ml PA-IIL solution before applying the mixture to the sections. The final concentration of L-fucose in solution ranged from 0.16 mM to 0.01 mM L-fucose.

To analyze the loss of L-fucose activity to block PA-IIL tissue binding in relation to storage time in an aqueous solution, L-fucose was dissolved in water and then stored for different durations (for 0, 40, 80, 160, 320 minutes as well as 12 and 24 h). After storage L-fucose was mixed with the PA-IIL solution and used to incubate human respiratory epithelial tissue samples as described above.

### Decrease of PA-IIL blocking efficacy by L-fucose dissolved in solution over time

The PA-IIL blocking efficacy of the aqueous solution comprising L-fucose was analyzed in relation to the duration of storage after dissolving the L-fucose in the aqueous solution. Maximum activity was detected when L-fucose was added to PA-IIL immediately after dissolving the same in water. Freshly prepared L-fucose solution had significant blocking activity which significantly decreased over time and was completely abolished after 24 hours storage time. Representative results are shown in Figures 1-5.

This proof-of-principle experiment shows that L-fucose has significant PA-IIL blocking efficacy which is however lost over a short period of time of less than 24 hours during storage in an aqueous solution.

## Claims

1. A composition comprising D-galactose and L-fucose for use in treating or preventing infections in an individual, wherein the composition is administered to the individual in solid form, preferably as a powder.

2. A composition comprising D-galactose and L-fucose for use in treating or preventing infections in an individual, wherein the composition is administered to the individual in liquid form and wherein the liquid composition is produced by mixing D-galactose and L-fucose in solid form with a liquid no longer than 20 hours before administering the composition to the individual.

3. A composition comprising D-galactose for use in treating or preventing infections in an individual, wherein treating comprises the administration of D-galactose and L-fucose according to one of claims 1 or 2.

4. A composition comprising L-fucose for use in treating or preventing infections in an individual, wherein treating comprises the administration of D-galactose and L-fucose according to one of claims 1 or 2.

5. The composition for use in treating or preventing infections in an individual according to any one of the preceding claims, wherein the composition is a liquid composition that is prepared by mixing D-galactose and L-fucose in solid form with a liquid to obtain the composition no longer than 12 hours before administering the composition to the individual, no longer than 6 hours, no longer than 3 hours, no longer than 1 hour or no longer than 30 minutes before administering the composition to the individual.

6. The composition for use in treating or preventing infections in an individual according to any one of the preceding claims, wherein D-galactose is the beta anomer and L-fucose is the alpha anomer.

7. The composition for use in treating or preventing infections in an individual according to any one of the preceding claims, wherein the relative concentration of D-galactose to L-fucose is in the range of 1:10000 to 10000:1, preferably 1:1.

8. The composition for use in treating or preventing infections in an individual according to any one of claims 1 or 3 to 7, wherein the composition is a solid composition, and wherein the quantity of D-galactose is in the range of 0.001 mg to 10 g per cm² application site and wherein the quantity of L-fucose is in the range of 0.001 mg to 10 g per cm² application site, preferably the quantity of D-galactose in the solid composition is 0.02 g per cm² application site and the quantity of L-fucose in the solid composition is 0.02 g per cm² application site.

9. The composition for use in treating or preventing infections in an individual according to any one of claims 2 to 7, wherein the composition is a liquid composition, and wherein the concentration of D-galactose in the liquid composition is in the range of 0.001 mM to 3.0 M and wherein the concentration of L-fucose in the liquid composition is in the range of 0.001 mM to 3.0 M, preferably the concentration of D-galactose in the liquid composition is 0.15 M and the concentration of L-fucose in the liquid composition is 0.15 M.

10. The composition for use in treating or preventing infections in an individual according to any one of the preceding claims, wherein the composition is inhaled and the dose of D-galactose per inhalation is in the range of 0.001 mg to 5.0 g and the dose of L-fucose per inhalation is in the range of 0.001 mg to 5.0 g, preferably the dose of D-galactose per inhalation is 0.1 g and the application dose of L-fucose per inhalation is 0.1 g.

11. The composition for use in treating or preventing infections in an individual according to any one of the preceding claims, wherein the infection is suspected to be caused by a microorganism expressing L-fucose- or D-galactose-binding lectins, for example *Pseudomonas aeruginosa.*

12. The composition for use in treating or preventing infections in an individual according to any one of the preceding claims, wherein the composition is to be administered topically.

13. The composition for use in treating or preventing infections in an individual according to any one of the preceding claims, wherein the infection is a respiratory tract infection, for example in patients suffering from cystic fibrosis, ear nose throat infection, for example otitis externa, skin infection, dermatitis, wound infection, urinary tract infection, catheter-related infection, osteomyelitis, implant-associated infection, eye infection, surgical site infection or joint infection.

14. The composition for use in treating or preventing infections in an individual according to any one of the preceding claims, wherein the composition is transferred into a different aggregate state or form of administration.

15. The composition for use in treating or preventing infections in an individual according to claim 14, wherein the composition is administered as a powder, an aerosol, a liquid composition of varying viscosities, for example a gel or an ointment or wherein administration of the composition comprises inhaling the composition.

16. A container comprising a solid composition according to any one of claims 1 and 3 to 15, preferably a powder, and an opening for administering the solid composition to a patient.

17. A container comprising two compartments and a means for dispensing the content of each compartment, wherein one compartment comprises a liquid and the other compartment comprises a solid composition according to any one of the preceding claims, and wherein the container comprises a means for mixing the solid composition with the liquid.

18. The container according to claim 17, wherein the mixing is carried out no longer than 20 hours before or during administering the composition.

19. A dressing, bandage or plaster comprising a composition according to any one of the preceding claims, which can be directly applied to the infected site.

20. A device such as an inhaler or nebulizer to deliver a composition according to any one of the preceding claims to the lungs of a patient.

## Amended claims

### Amended claims in accordance with Rule 137(2) EPC.

1. A composition comprising D-galactose and L-fucose for use in treating or preventing infections in an individual, wherein the composition is administered to the individual in solid form, preferably as a powder.

2. A composition comprising D-galactose and L-fucose for use in treating or preventing infections in an individual, wherein the composition is administered to the individual in liquid form and wherein the liquid composition is produced by mixing D-galactose and L-fucose in solid form with a liquid no longer than 3 hours before administering the composition to the individual.

3. A composition comprising D-galactose for use in treating or preventing infections in an individual, wherein treating comprises the administration of D-galactose and L-fucose according to one of claims 1 or 2.

4. A composition comprising L-fucose for use in treating or preventing infections in an individual, wherein treating comprises the administration of D-galactose and L-fucose according to one of claims 1 or 2.

5. The composition for use in treating or preventing infections in an individual according to any one of the preceding claims, wherein the composition is a liquid composition that is prepared by mixing D-galactose and L-fucose in solid form with a liquid to obtain the composition no longer than 1 hour or no longer than 30 minutes before administering the composition to the individual.

6. The composition for use in treating or preventing infections in an individual according to any one of the preceding claims, wherein D-galactose is the beta anomer and L-fucose is the alpha anomer.

7. The composition for use in treating or preventing infections in an individual according to any one of the preceding claims, wherein the relative concentration of D-galactose to L-fucose is in the range of 1:10000 to 10000:1, preferably 1:1.

8. The composition for use in treating or preventing infections in an individual according to any one of claims 1 or 3 to 7, wherein the composition is a solid composition, and wherein the quantity of D-galactose is in the range of 0.001 mg to 10 g per cm² application site and wherein the quantity of L-fucose is in the range of 0.001 mg to 10 g per cm² application site, preferably the quantity of D-galactose in the solid composition is 0.02 g per cm² application site and the quantity of L-fucose in the solid composition is 0.02 g per cm² application site.

9. The composition for use in treating or preventing infections in an individual according to any one of claims 2 to 7, wherein the composition is a liquid composition, and wherein the concentration of D-galactose in the liquid composition is in the range of 0.001 mM to 3.0 M and wherein the concentration of L-fucose in the liquid composition is in the range of 0.001 mM to 3.0 M, preferably the concentration of D-galactose in the liquid composition is 0.15 M and the concentration of L-fucose in the liquid composition is 0.15 M.

10. The composition for use in treating or preventing infections in an individual according to any one of the preceding claims, wherein the composition is inhaled and the dose of D-galactose per inhalation is in the range of 0.001 mg to 5.0 g and the dose of L-fucose per inhalation is in the range of 0.001 mg to 5.0 g, preferably the dose of D-galactose per inhalation is 0.1 g and the application dose of L-fucose per inhalation is 0.1 g.

11. The composition for use in treating or preventing infections in an individual according to any one of the preceding claims, wherein the infection is suspected to be caused by a microorganism expressing L-fucose- or D-galactose-binding lectins, for example *Pseudomonas aeruginosa.*

12. The composition for use in treating or preventing infections in an individual according to any one of the preceding claims, wherein the composition is to be administered topically.

13. The composition for use in treating or preventing infections in an individual according to any one of the preceding claims, wherein the infection is a respiratory tract infection, for example in patients suffering from cystic fibrosis, ear nose throat infection, for example otitis externa, skin infection, dermatitis, wound infection, urinary tract infection, catheter-related infection, osteomyelitis, implant-associated infection, eye infection, surgical site infection or joint infection.

14. The composition for use in treating or preventing infections in an individual according to any one of the preceding claims, wherein the composition is transferred into a different aggregate state or form of administration, wherein the composition is administered as a powder, an aerosol, a liquid composition of varying viscosities, for example a gel or an ointment or wherein administration of the composition comprises inhaling the composition.

15. A container comprising a solid composition according to any one of claims 1 and 3 to 14, preferably a powder, and an opening for administering the solid composition to a patient.

16. A container comprising two compartments and a means for dispensing the content of each compartment, wherein one compartment comprises a liquid and the other compartment comprises a solid composition according to any one of the preceding claims, and wherein the container comprises a means for mixing the solid composition with the liquid.

17. The container according to claim 16, wherein the mixing is carried out no longer than 3 hours before or during administering the composition.

18. A dressing, bandage or plaster comprising a composition according to any one of the preceding claims, which can be directly applied to the infected site.

19. A device such as an inhaler or nebulizer to deliver a composition according to any one of the preceding claims to the lungs of a patient.
